(19) Europäisches Patentamt
European Patent Office
Office européen des brevets

(11) Publication number: **0 178 388**
**A1**

(12) # EUROPEAN PATENT APPLICATION

(21) Application number: 85108951.6

(22) Date of filing: 17.07.85

(51) Int. Cl.⁴: C 07 D 401/04
A 61 K 31/495

(30) Priority: 11.10.84 JP 213029/84

(43) Date of publication of application: 23.04.86 Bulletin 86/17

(84) Designated Contracting States: AT BE CH DE FR GB IT LI LU NL SE

(71) Applicant: KYORIN PHARMACEUTICAL CO., LTD.
No. 5, Kanda Surugadai 2-chome
Chiyoda-ku Tokyo(JP)

(72) Inventor: Irikura, Tsutomu
No. 10-28, Ooizumigakuen-cho 7-chome
Nerima-ku Tokyo(JP)

(72) Inventor: Suzue, Seigo
No. 13-4, Aoba 4-chome
Kuki-shi Saitama-ken(JP)

(72) Inventor: Hirai, Keiji
No. 1-2-512, Aoba 1-chome
Kuki-shi Saitama-ken(JP)

(72) Inventor: Ishizaki, Takayoshi
No. 9-6, Sakurada 4-chome Washimiya-machi
Kitakatsushika-gun Saitama-ken(JP)

(74) Representative: Patentanwälte TER MEER - MÜLLER - STEINMEISTER
Mauerkircherstrasse 45
D-8000 München 80(DE)

(54) **Quinolinecarboxylic acid derivatives.**

(57) Quinolonecarboxylic acid derivatives of the following formula,

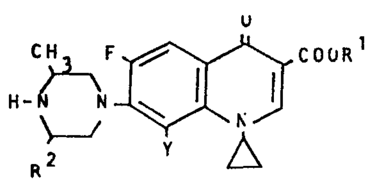

wherein $R^1$ is hydrogen atom or lower alkyl group, $R^2$ is hydrogen atom or methyl group and Y is chlorine or fluorine atom, the hydrates and pharmaceutically acceptable salts thereof, are useful as an antibacterial agent.

0178388

Title of the invention:

Quinolonecarboxylic acid derivetives

Detailed description of the invention:

This invention is concerned with certain novel useful quinolonecarboxylic acid derivatives of the following formula (I),

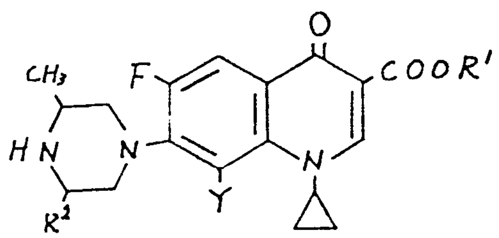

(I)

wherein $R^1$ is hydrogen atom or lower alkyl group, $R^2$ is hydrogen atom or methyl group and Y is chlorine or fluorine atom, the hydrates or pharmaceutically acceptable salts thereof, with a process for their preparation, and with use as antibacterial agent.

The present inventors have found that new compounds

according to the present invention show better activity and broader spectrum both in vitro and in vivo against Gram-negative and Gram-positive bacteria than the previously known quinolonecarboxylic acid derivatives. The present compounds are well absorbed when given orally and very well tolerated on oral or parenteral administration in animals.

The present compounds, therefore, are active at low doses against both Gram-positive and Gram-negative bacteria and thus constitute valuable agents for the treatment of infectious human, animal or plant diseases.

The compounds of the formula (I) are synthesized by reacting a compound of the formula (II),

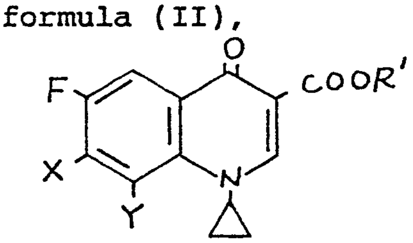

(II)

wherein X is halogen atom, $R^1$ and Y are the same as defined above, with a compound of the formula (III),

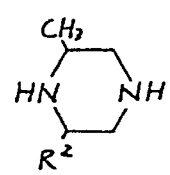

(III)

wherein $R^2$ is the same as defined above. The reaction is preferably carried out by heating the two reactants in a solvent such as water, alcohols, acetonitrile, dimethylformamide, dimethyl sulfoxide, hexamethylphosphoric triamide, pyridine, picoline and the like or in absence of the solvent at a temperature of room to 200°C, preferably 60 to 160°C, more preferably 60 to 120°C for 1 to several hours. It is desirable

that a slight excess (2 to 5 moles) of the secondary amine of the formula (III) is used per mole of the compound of the formula (II) and a solvent is used such that the mixture remains homogeneous after dissolution of the compound of the formula (II) (2 to 20-fold volume of the compound of the formula (III)).

When $R^1$ in the formula (II) is lower alkyl, the reaction product (carboxylic ester) is saponified to the corresponding carboxylic acid by the usual manner.

Furthermore, the compounds of the formula (I) can be converted, if desired, to the pharmaceutically acceptable piperazinium salts or carboxylic acid metal salts by treatment with acid or alkali. The acid may be organic or inorganic acids such as, for example, hydrochloric acid, sulfuric acid, methanesulfonic acid, phosphoric acid, acetic acid and lactic acid. The carboxylic acid metal salts may be, for example, sodium, potassium, magnesium, calcium, aluminum, cerium, chromium, cobalt, copper, iron, zinc, and silver salts.

The compound of the formula (I), hydrates and salts thereof may be used as medicines in the conventional form of pharmaceutical preparations, which may be, for example, tablets, capsules, powder, ointments, supositories, injections or eye drops, suitable for peroral, parenteral, enteral or local administration.

The following examples will further illustrate the invention without, however, limiting it thereto.

Example 1 Ethyl 1-cyclopropyl-6,8-difluoro-1,4-dihydro-7-(3-methyl-1-piperazinyl)-4-oxoquinoline-3-carboxylate

To a hot solution (80°C) of 2-methylpiperazine (0.39 g) in dimethyl sulfoxide (5 ml) was added ethyl 1-cyclopropyl-6,7,8-trifluoro-1,4-dihydro-4-oxoquinoline-3-carboxylate (0.30 g) and the mixture was stirred at 80-90°C for 3 hours. After cooling, the reaction mixture was poured into ice-water (50 ml) and extracted 3 times with chloroform (each 50 ml). The chloroform layer was extracted 5 times with diluted hydrochloric acid solution (each 50 ml). To the acid extract was added chloroform. The mixture was alkalized (pH 10-11) by adding aqueous sodium hydroxide solution. The organic layer was separated as soon as possible and washed with water, dried over anhydrous sodium sulfate and concentrated.

The residue was recrystallized from ethyl acetate-n-hexane to give the title compound (0.29 g), mp 155-157°C.

Analysis (%) for $C_{20}H_{23}F_{2}N_{3}O_{3}$, Calcd. (Found): C, 61.37 (61.21); H, 5.92 (5.95); N, 10.74 (10.69).

Example 2 1-Cyclopropyl-6,8-difluoro-1,4-dihydro-7-(3-methyl-1-piperazinyl)-4-oxoquinoline-3-carboxylic acid

A mixture of ethyl 1-cyclopropyl-6,8-difluoro-1,4-dihydro-7-(3-methyl-1-piperazinyl)-4-oxoquinoline-3-carboxylate (0.19 g) and 1N sodium hydroxide solution (9 ml) was stirred at 100°C for 50 minutes. After cooling, the mixture was neutralized (pH 7) by adding acetic acid and concentrated to dryness. Water (2 ml) was added to,the residue. The mixture was allowed to stand overnight in a refrigerator. The precipitate was collected by filtration and recrystallized from methanol to give the title

compound (25 mg), mp 227-228°C.

Analysis (%) for $C_{18}H_{19}F_2N_3O_3 \cdot 1/2\ H_2O$, Calcd. (Found): C, 58.06 (58.22); H, 5.41 (5.13); N, 11.28 (11.30).

Example 3 Ethyl 1-cyclopropyl-6,8-difluoro-1,4-dihydro-7-(3,5-dimetyl-1-piperazinyl)-4-oxoquinoline-3-carboxylate

To a hot solution (80°C) of 2,6-dimethylpiperazine (0.44 g) in dimethyl sulfoxide (5 ml) was added ethyl 1-cyclopropyl-6,7,8-trifluoro-1,4-dihydro-4-oxoquinoline-3-carboxylate (0.30 g) and the mixture was stirred at 80-90°C for 3 hours. After cooling, the reaction mixture was poured into ice-water (50 ml) and extracted 3 times with chloroform (each 50 ml). The chloroform layer was extracted 5 times with diluted hydrochloric acid solution (each 50 ml). To the acid extract was added chloroform. The mixture was alkalized (pH 10-11) by adding aqueous sodium hydroxide solution. The organic layer was separated as soon as possible and washed with water, dried over anhydrous sodium sulfate and concentrated. The residue was recrystallized from ethyl acetate-n-hexane to give the title compound (0.31 g), mp 171-172°C.

Analysis for $C_{21}H_{25}F_2N_3O_3$, Calcd. (Found): C, 62.21 (62.10); H, 6.21 (6.23); N, 10.36 (10.30).

Example 4 1-Cyclopropyl-6,8-difluoro-1,4-dihydro-7-(3,5-dimethyl-1-piperazinyl)-4-oxoquinoline-3-carboxylic acid

A mixture of ethyl 1-cyclopropyl-6,8-difluoro-7-(3,5-dimethyl-1-piperazinyl)-4-oxoquinoline-3-carboxylate (0.23 g) and

1N sodium hydroxide solution (9 ml) was stirred at 100°C for 50 minutes. After cooling, the mixture was neutralized (pH 7) by adding acetic acid and concentrated to dryness. Water (2 ml) was added to the residue. The mixture was allowed to stand overnight in a refrigerator. The precipitate was collected by filtration and recrystallized from methanol to give the title compound (29 mg), mp 250-251°C.

Analysis (%) for $C_{19}H_{21}F_2N_3O_3 \cdot 1/2\ H_2O$, Calcd. (Found): C, 59.06 (58.68); H, 5.74 (5.38); N, 10.84 (10.74).

Example 5 8-Chloro-1-cyclopropyl-6-fluoro-1,4-dihydro-7-(3-methyl-1-piperazinyl)-4-oxoquinoline-3-carboxylic acid hydrochloride

A mixture of 2-methylpiperazine (70 mg) and 8-chloro-1-cyclopropyl-6,7-difluoro-1,4-dihydro-4-oxoquinoline-3-carboxylic acid (50 mg) in dimethyl sulfoxide (1 ml) was stirred at 65-70°C for 1 hour. After cooling, the reaction mixture was diluted with diluted hydrochloric acid solution to give the acid mixture (pH<1). The mixture was concentrated to dryness. The residue was recrystallized from ethanol-water to give the title compound (32 mg), mp 248-255°C (decompd.).

Analysis (%) for $C_{18}H_{19}ClFN_3O_3 \cdot HCl \cdot 1/3\ H_2O$, Calcd. (Found): C, 51.20 (51.17); H, 4.93 (4.83); N, 9.95 (10.01).

Example 6 8-Chloro-1-cyclopropyl-6-fluoro-1,4-dihydro-7-(3,5-dimethyl-1-piperazinyl)-4-oxoquinoline-3-carboxylic acid and its hydrochloride

A mixture of 2,6-dimethylpiperazine (155 mg) and 8-chloro-1-cyclopropyl-6,7-difluoro-1,4-dihydro-4-oxoquinoline-3-carboxylic

acid (76 mg) in dimethyl sulfoxide (1.5 ml) was stirred at 49-60°C for 55 minutes. The reaction mixture was concentrated to dryness. The residue was dissolved in diluted aqueous sodium hydroxide solution, then the solution was neutralized by adding acetic acid. The precipitate was collected by filtration to give the title compound (19 mg) as yellow prisms, mp 221-223.5°C (decompd.).

Analysis (%) for $C_{19}H_{21}ClFN_3O_3 \cdot 3/4\ H_2O$, Calcd. (Found): C, 56.02 (55.85); H, 5.57 (5.20); N, 10.32 (10.24).

On the other hand, the cooled filtrate was acidfied (pH<1) by adding concentrated hydrochloric acid to deposit its hydrochloride (19 mg), which was recrystallized from methanol-dichloromethane to give colorless prisms, mp 243-247.5°C (decompd.).

Analysis (%) for $C_{19}H_{21}ClFN_3O_3 \cdot HCl \cdot 1/2\ H_2O$, Calcd. (Found): C, 51.95 (52.02); H, 5.28 (5.18); N, 9.56 (9.42).

Example 7 8-Chloro-1-cyclopropyl-6-fluoro-1,4-dihydro-7-(3-methyl-1-piperazinyl)-4-oxoquinoline-3-carboxylic acid

A mixture of 2-methylpiperazine (2.67 g) and 8-chloro-1-cyclopropyl-6,7-difluoro-1,4-dihydro-4-oxoquinoline-3-carboxylic acid (2.00 g) in dimethyl sulfoxide (13 ml) was stirred at 60-65°C for an hour and concentrated to dryness. Water (25 ml) was added to the residue. After adjusted to pH 9-10 by adding 1N-sodium hydroxide solution, the mixture was warmed to give clear solution and neutralized (pH 7) by the addition of acetic acid. After cooling, the precipitate was collected by filtration and

recrystallized from 200 ml of chloroform-methanol (1 : 1) with Norite to give pale brown prisms (1.18 g), mp 246-248°C (decompd.).

Analysis (%) for $C_{18}H_{19}ClFN_3O_3 \cdot 1/2\ H_2O$, Calcd. (Found): C, 55.60 (55.39); H, 5.18 (4.83); N, 10.81 (10.72).

Experiment 1 In vitro antibacterial activity

The in vitro antibacterial activity has been investigated, the minimun inhibitory concentration (MIC) was determined in accordance with the method recommended by Japan Society of Chemotherapy. The results of the test are shown in Table 1-a and 1-b.

Table 1-a In vitro antibacterial activity (standard strain)

| Organism ($10^6$ cells/ml) | Gram | MIC (μg/ml) | | | | | |
|---|---|---|---|---|---|---|---|
| | | Exp.2 | Exp.4 | Exp.5 | Exp.6 | Exp.7 | CFLX |
| Bacillus subtilis PCI 219 | + | 0.05 | 0.05 | 0.025 | 0.05 | - | 0.05 |
| Staphylococcus aureus 209 P | + | 0.20 | 0.20 | 0.10 | 0.20 | 0.10 | 0.39 |
| S. aureus IID 670 (Terajima) | + | 0.20 | 0.20 | 0.10 | 0.20 | - | 0.39 |
| S. epidermidis IID 866 | + | 0.20 | 0.39 | 0.10 | 0.20 | 0.10 | 0.39 |
| Streptococcus pyogenes S-8 | + | 0.78 | - | 0.39 | 0.39 | - | - |
| S. pyogenes IID 692 | + | 0.78 | - | 0.78 | 0.78 | - | - |
| S. pneumoniae IID 552 | + | 0.78 | - | 0.78 | 0.78 | - | - |
| S. faecalis IID 682 | + | 0.78 | - | 0.39 | 0.78 | - | - |
| Escherichia coli NIHJ JC-2 | - | ≦0.006 | ≦0.025 | ≦0.006 | 0.05 | - | ≦0.006 |
| E. coli ATCC 10536 | - | ≦0.006 | ≦0.006 | 0.0125 | 0.025 | 0.0125 | 0.0125 |
| Proteus vulgaris IFO 3167 | - | 0.0125 | 0.025 | 0.0125 | 0.05 | - | ≦0.006 |
| P. mirabilis IID 994 | - | 0.025 | 0.10 | 0.025 | 0.10 | - | 0.0125 |
| P. morganii IID 602 | - | 0.05 | 0.10 | 0.10 | 0.20 | - | 0.05 |
| Klebsiella pneumoniae I-220S | - | 0.05 | - | 0.05 | 0.20 | - | 0.05 |
| K. pneumoniae KY(GN)6445 | - | 0.0125 | 0.025 | 0.025 | 0.05 | 0.025 | 0.025 |
| Enterobacter cloacae IID 977 | - | 0.05 | 0.05 | 0.05 | 0.20 | - | 0.05 |
| Citrobacter freundii IID 976 | - | 0.0125 | 0.025 | 0.025 | 0.10 | - | ≦0.006 |
| Serratia marcescens IID 618 | - | 0.05 | 0.10 | 0.05 | 0.20 | 0.05 | 0.39 |
| Shigella sonnei IID 969 | - | 0.0125 | 0.0125 | 0.0125 | 0.05 | - | ≦0.006 |
| Salmonella enteritidis IID 604 | - | 0.025 | 0.05 | 0.025 | 0.10 | - | 0.025 |
| Pseudomonas aeruginosa V-1 | - | 0.20 | 0.39 | 0.78 | 1.56 | - | 0.20 |
| P. aeruginosa IFO 12689 | - | 0.78 | 1.56 | 1.56 | 3.13 | 0.78 | 0.39 |
| Yersinia enterocolitica IID 981 | - | 0.05 | 0.10 | 0.05 | 0.20 | - | 0.05 |
| Acinetobacter anitratus IID 876 | - | 0.006 | 0.006 | 0.05 | 0.10 | - | 0.025 |
| Alcaligenes faecalis 0114002 | - | 0.20 | 0.20 | 0.39 | 0.78 | - | 0.39 |
| Bacteroides fragilis GM 7000 | - | 1.56 | 3.13 | 0.39 | - | - | 3.13 |
| B. fragilis 0558 | - | 0.78 | 1.56 | 0.20 | - | - | 3.13 |
| B. fragilis 25285 | - | 0.78 | 1.56 | 0.39 | - | - | 3.13 |
| B. distasonis 8503 | - | 1.56 | 3.13 | - | - | - | - |
| B. thetaiotaomicron (0661) | - | 3.13 | 6.25 | 1.56 | - | - | 3.13 |
| Fusobacterium necrophorum S-45 | - | - | - | 0.39 | - | - | - |
| F. varium KYA (ATCC) 8501 | - | 6.25 | 12.5 | 3.13 | - | - | 12.5 |
| Eubacterium lentum GAI 5242 | + | - | - | 0.39 | - | - | - |
| Propionibacterium acens 11828 | + | - | - | 0.39 | - | - | - |
| Peptococcus magnus KY 017 | + | - | - | 0.10 | - | - | - |
| Clostridium difficile I-E | + | 6.25 | 12.5 | 3.13 | - | - | - |
| C. perfringens KYA (ATCC) 13123 | + | 0.78 | 1.56 | 0.78 | - | - | 0.39 |
| C. ramosum | + | - | - | 3.13 | - | - | 3.13 |
| Peptostreptococcus anaerobius KYA 27337 | + | 1.56 | 3.13 | 0.39 | - | - | 0.78 |
| Pst. micros UPI 5464-1 | + | 0.20 | 0.39 | 0.20 | - | - | - |
| Veillonella parvula KYA 10790 | - | 0.20 | 0.39 | 0.20 | - | - | - |

CFLX: 1-Cyclopropyl-6-fluoro-1,4-dihydro-4-oxo-7-(1-piperazinyl)-quinoline-3-carboxylic acid (Ciprofloxacin)

Table 1-b In vitro antibacterial activity
(clinical isolates)

| Organism ($10^6$ cells/ml) | Gram | MIC (μg/ml) | | |
|---|---|---|---|---|
| | | Exp.2 | Exp.5 | CFLX |
| S. pneumoniae 15 | + | 0.78 | 0.78 | 1.56 |
| S. pneumoniae 2054 | | 1.56 | 0.78 | 1.56 |
| S. pneumoniae 2950 | | 1.56 | 0.78 | 1.56 |
| S. pneumoniae 3227 | | 1.56 | 0.78 | 1.56 |
| S. pyogenes 3130 | + | 0.78 | 0.39 | 0.39 |
| S. pyogenes 3102 | | 0.78 | 0.39 | 0.39 |
| S. pyogenes 3107 | | 0.78 | 0.39 | 0.39 |
| S. pyogenes 4340 | | 0.78 | 0.39 | 0.39 |
| S. pyogenes 4372 | | 0.78 | 0.39 | 0.39 |
| S. agalactiae 4394 | + | 1.56 | 0.78 | 1.56 |
| S. agalactiae 4049 | | 1.56 | 0.78 | 1.56 |
| S. agalactiae 4342 | | 0.78 | 0.78 | 0.78 |
| S. agalactiae 4470 | | 0.78 | 0.78 | 0.78 |
| S. agalactiae 4368 | | 0.78 | 0.39 | 0.39 |
| S. agalactiae 4468 | | 0.78 | 0.39 | 0.78 |
| S. faecalis 49 | + | 0.78 | 0.78 | 0.78 |
| S. faecalis 214 | | 0.78 | 0.78 | 0.78 |
| S. faecalis 401 | | 1.56 | 0.78 | 1.56 |
| S. faecalis 402 | | 1.56 | 0.78 | 1.56 |

CFLX: 1-Cyclopropyl-6-fluoro-1,4-dihydro-4-oxo-7-(1-piperazinyl)quinoline-3-carboxylic acid (Ciprofloxacin)

Experiment 2 In vivo antibacterial activity against systemic infection in mice (ICR-S)

The infections were produced by intraperitoneal infection of a suspension of the bacterial culture corresponding to the germ studied. The product was administered orally at 1 hour after the infection. The 50% effective dose ($ED_{50}$) which protects 50% of the animals from death caused by the infection was determined from the relation between dosage and survival rate.

The efficacy of the compounds of this invention are shown in Table 2 together with the known compound.

The present compounds were more effective than the reference substance.

Table 2 In vivo antibacterial activity against systemic infection in mice (ICR-S)

(part 1)

| Compound | E. coli ML4707 | |
|---|---|---|
| | MIC (μg/ml) | $ED_{50}$ (mg/kg) |
| Example 2 | 0.0125 | 0.09 |
| Example 4 | 0.0125 | 0.13 |
| CFLX | 0.0125 | 0.67 |

Challenge dose: 6.0 x $10^6$ cfu/mouse

(part 2)

| Compound | MIC (μg/ml) | E. coli ML4707 ED$_{50}$ (mg/kg) |
| --- | --- | --- |
| Example 2 | 0.0125 | 0.17 |
| Example 5 | 0.0125 | 0.12 |
| CFLX | 0.0125 | 0.75 |

Challenge dose: 8.3 x $10^6$ cfu/mouse

(part 3)

| Compound | MIC (μg/ml) | P. aeruginosa IID 1210 ED$_{50}$ (mg/kg) |
| --- | --- | --- |
| Example 5 | 0.78 | 3.2 |
| CFLX | 0.39 | 8.0 |

Challenge dose: 2.5 x $10^5$ cfu/mouse

(part 4)

| Compound | MIC (μg/ml) | S. pneumoniae S-4288 ED$_{50}$ (mg/kg) |
|---|---|---|
| Example 7 | 0.78 | 15.6 |
| CFLX | 1.56 | >100 |

Challenge dose: 2.5 x $10^3$ cfu/mouse

(part 5)

| Compound | MIC (μg/ml) | S. aureus Smith ED$_{50}$ (mg/kg) |
|---|---|---|
| Example 5 | 0.10 | 0.89 |
| CFLX | 0.39 | 12.8 |

Challenge dose: 3.5 x $10^5$ cfu/mouse

CFLX: 1-Cyclopropyl-6-fluoro-1,4-dihydro-4-oxo-7-(1-piperazinyl)quinoline-3-carboxylic acid

What is claimed is:

1. A compound of the formula (I);

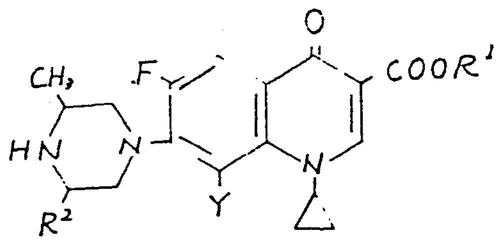

(I)

wherein $R^1$ is hydrogen atom or lower alkyl group, $R^2$ is hydrogen atom or methyl group and Y is chlorine or fluorine atom; the hydrates or the pharmaceutically acceptable acid addition or alkali salts thereof.

2. A process for the preparation of a compound of the formula (I) specified in claim 1, which comprises condensing a compound of the formula (II);

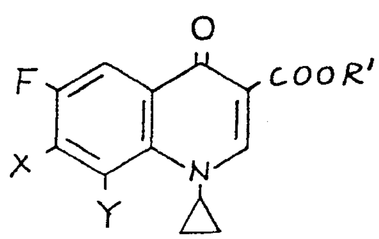

(II)

wherein $R^1$ is hydrogen atom or lower alkyl group, X is halogen atom and Y is chlorine or fluorine atom ; with a secondary amine of the formula (III);

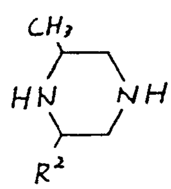

(III)

wherein $R^2$ is hydrogen atom or methyl group.

3. A process for the preparation of a compound of the formula (I) specified in claim 1, in which $R^1$ is a hydrogen atom, which comprises saponifying a compound of the formula (I-a),

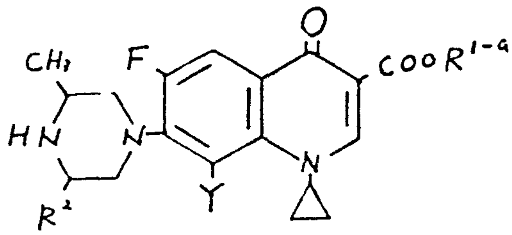

(I-a)

wherein $R^{1-a}$ is lower alkyl, $R^2$ is hydrogen atom or methyl group and Y is chlorine or fluorine atom.

4. An antibacterial pharmaceutical composition comprising at least one compound according to claim 1 and an inert pharmaceutical acceptable carrier.

What is claimed is:

1. A process for the preparation of a compound of the formula (I)

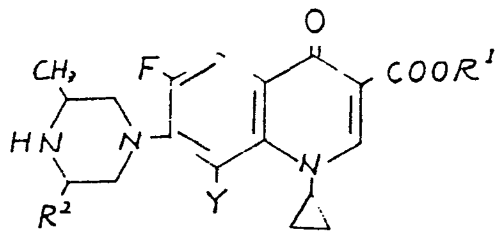

(I)

wherein $R^1$ is hydrogen atom or lower alkyl group, $R^2$ is hydrogen atom or methyl group and Y is chlorine or fluorine atom; the hydrates or the pharmaceutically acceptable acid addition or alkyli salts thereof, which comprises condensing a compound of the formula (II);

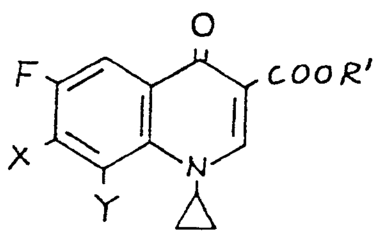

(II)

wherein $R^1$ is hydrogen atom or lower alkyl group, X is halogen and Y is chlorine or fluorine atom; with a secondary amine of the formula (III);

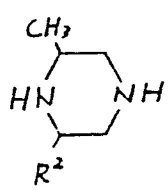

(III)

wherein $R^2$ is hydrogen atom or methyl group.

2. A process for the preparation of a compound of the formula (I);

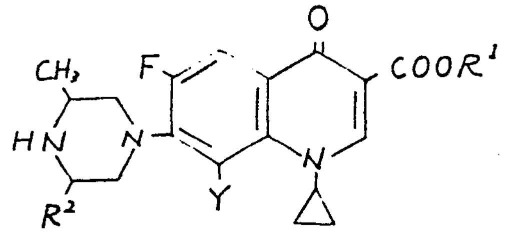

(I)

wherein $R^1$ is hydrogen atom or lower alkyl group, $R^2$ is hydrogen atom or methyl group and Y is chlorine or fluorine atom; the hydrates or the pharmeceutically acceptable acid addition or alkali salts thereof, in which $R^1$ is a hydrogen atom, which comprises saponifying a compound of the formula (I-a),

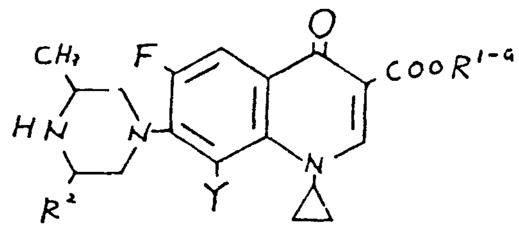

(I-a)

wherein $R^{1-a}$ is lower alkyl, $R^2$ is hydrogen atom or methyl group and Y is chlorine or fluorine atom.

European Patent Office

# EUROPEAN SEARCH REPORT

0178388
Application number

EP 85 10 8951

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 113 093 (BAYER) * Claim 1; page 13, lines 7-21 * | 1,4 | C 07 D 401/04<br>A 61 K 31/495 |
| | --- | | |
| Y | BE-A- 899 399 (KYORIN PHARMACEUTICAL CO., LTD.) * Claim 1; pages 10-12 * | 1,4 | |
| | --- | | |
| P,X | EP-A-0 126 355 (BAYER) * Page 26, examples 3-5; claims 1,10 * | 1,4 | |
| | ----- | | |
| | | | TECHNICAL FIELDS SEARCHED (Int. Cl.4) |
| | | | C 07 D 401/00<br>A 61 K 31/00 |

The present search report has been drawn up for all claims

| Place of search | Date of completion of the search | Examiner |
|---|---|---|
| THE HAGUE | 14-01-1986 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82